# EUROPÄISCHE PATENTANMELDUNG

(11) **EP 0 730 878 A2**
(43) Veröffentlichungstag der Anmeldung: **11.09.1996**
(21) Anmeldenummer: 96810111.3
(22) Anmeldetag: 29.02.1996
(51) Int. Cl.: A61M 16/10

(54) **Vorrichtung zur Behandlung von Atemluft**

(30) Priorität: 09.03.1995 CH 671/95
(71) Anmelder: Medisize BV, 2181 AB Hillegom (NL)
(72) Erfinder: Van den Bruinhorst, Willem, 3628 ES Kockengen (NL); Lippers, Herman, 1052 MA Amsterdam (NL)
(74) Vertreter: Hepp, Dieter

(57) **Zusammenfassung**

Die Erfindung betrifft eine Vorrichtung zur Atemluft-Behandlung, welche in den Luftschlauch eingekoppelt wird, der den Patienten mit einer Beatmungsmaschine verbindet. In der Regel handelt es sich um einen sogenannten Wärme-Feuchtigkeits-Tauscher, der von Zeit zu Zeit ausgewechselt werden muss. Das Gehäuse (1) der Vorrichtung weist zwei Anschlussöffnungen (5, 6) auf, welche im Betrieb mit je einem Schlauch verbunden sind. Um diese Vorrichtung im Bedarfsfall zu ersetzen, müssen die Schläuche entfernt und anschliessend auf die Anschlussrohre (15) der Ersatz-Vorrichtung aufgebracht werden. Dies muss mit Vorsicht geschehen, da die Vorrichtung ganz in der Nähe des Patienten angebracht wird und unkontrollierte Bewegungen des Schläuche dem Patienten Schmerzen verursachen können. Um das sichere Halten des Gehäuses während der Montage der Schläuche zu ermöglichen wird vorgeschlagen, an der Aussenfläche der Gehäusewand (3) eine umlaufende Greifrille (8) vorzusehen. Dies ist ohne aufwendige Geissformen möglich, indem wenigstens ein separat gefertigter Greifring (9) auf die Gehäusewand (3) aufgeschoben wird.

## Beschreibung

Die Erfindung betrifft eine Vorrichtung zur Behandlung von Atemluft gemäss dem Oberbegriff von Anspruch 1. Solche Vorrichtungen dienen zur Behandlung von Luft, welche durch einen Schlauch direkt in die Atemwege eines Patienten geleitet wird. Diese Art der direkten Luftzufuhr wird häufig angewendet, wenn Patienten über einen längeren Zeitraum hinweg künstlich beatmet werden müssen. Der mit einer Beatmungsmaschine gekoppelte Luftschlauch wird dabei direkt in die Luftröhre des Patienten eingeführt. Um Auswirkungen wie z.B. Hustenreiz oder das Austrocknen der Schleimhäute zu verhindern, muss die zugeführt Atemluft besonders behandelt werden. Dies ist notwendig, weil die Nasen- und Rachenschleimhäute, welche diese Aufgabe normalerweise übernehmen, bei dieser Beamtungsart umgangen werden. Bei der Behandlung kann es um das Erwärmen, Befeuchten oder Reinigen der zugeführten Atemluft gehen.

Bevorzugt wird dazu eine passiv arbeitende Vorrichtung verwendet, welche ganz in der Nähe des Patienten in den Luftschlauch eingekoppelt wird. Es handelt sich um ein Gehäuse mit wenigstens zwei Anschlussöffnungen, von denen eine mit dem Patienten und die andere mit einer Beatmungsmaschine verbunden wird. In dem Gehäuse ist ein Behandlungs-Einsatz angeordnet, dessen Aufbau von der Art der gewünschten Atemluft-Behandlung abhängt. In der Regel handelt es sich um einen sogenannten Wärme-Feuchtigkeits-Tauscher, der beim Ausatmen die in der ausgeatmeten Luft enthaltene Wärme und Feuchtigkeit aufnimmt und diese beim Einatmen wieder an das von der Beatmungsmaschine zugeführte Beatmungsgas abgibt. Es kann sich jedoch auch um einen einfachen Filter-Einsatz oder die Kombination eines Filters mit einem Wärme-Feuchtigkeits-Tauscher handeln.

Der Behandlungs-Einsatz der Vorrichtung muss von Zeit zu Zeit ausgewechselt werden, z.B. wenn er durch Lungen-Auswurf des Patienten verunreinigt ist. Die Behandlungs-Vorrichtung wird in der Regel als Einweg-Vorrichtung verwendet, das heisst, der Behandlungs-Einsatz wird mitsamt seinem Gehäuse entfernt und durch ein neues Gehäuse mit einem neuen Behandlungs-Einsatz ersetzt. Aber selbst wenn das Gehäuse der Vorrichtung wiederverwendet werden soll, so muss es dennoch zunächst von den Schläuchen des Patienten und der Beatmungsmaschine entfernt und anschliessend, nach dem Ersetzen des Behandlungs-Einsatzes erneut angeschlossen werden. Beim Anschliessen der Schläuche während der Montage, wie auch beim Entfernen der Schläuche während der Demontage der Vorrichtung, muss vorsichtig vorgegangen werden, da unkontrollierte Bewegungen der Schläuche dem Patienten Schmerzen verursachen können. Die bekannten Vorrichtungen haben in diesem Zusammenhang den Nachteil, dass sie eine im wesentlichen glatte Aussenfläche aufweisen und sich daher nicht besonders gut greifen lassen. Da zum Anbringen und Entfernen von Schläuchen ein gewisser Kraftaufwand notwendig ist, ist es für eine gut kontrollierte Montage der Schläuche von Vorteil, wenn die Form des Vorrichtungs-Gehäuses einen sicheren Haltegriff ermöglicht.

Es ist daher eine Aufgabe der Erfindung, das Gehäuse einer Vorrichtung zur Atemluft-Behandlung so zu gestalten, dass es sich leicht und sicher greifen lässt und gleichzeitig eine einfache Herstellung des Gehäuses möglich ist. Diese Aufgabe wird erfindungsgemäss durch eine Vorrichtung mit den Merkmalen von Anspruch 1 gelöst.

Es hat sich gezeigt, dass sich das Gehäuse der Vorrichtung besonders leicht und sicher greifen lässt, wenn dieses eine entlang dem Umfang seiner Gehäusewand verlaufende Greifrille aufweist. Die umlaufende Rille hat den Vorteil, dass sich die Vorrichtung in jeder Lage problemlos greifen lässt. In der Regel liegen die im Gehäuseboden und im Gehäusedeckel vorgesehen Anschlussöffnungen auf einer gemeinsamen Achse, so dass das Gehäuse im wesentlichen die Form eines geraden Rohrstücks hat, welches beidseitig an je einen Schlauch angeschlossen werden muss. Im Bereich der umlaufenden Greifrille kann das Gehäuse von der Seite her sicher gefasst werden, so dass ein Abrutschen praktisch ausgeschlossen ist und die Schläuche sicher mit den Anschlussöffnungen verbunden werden können.

Die einstückige Herstellung einer Gehäusewand, deren Aussenfläche eine umlaufende Greifrille aufweist, bringt jedoch mehrere Nachteile mit sich: Da die Form der Innenfläche des Gehäuses durch die Form des verwendeten Behandlungs-Einsatzes vorgegeben ist, kann zum Erzeugen der Greifrille nur die Aussenfläche verändert werden. Dies bedeutet, dass die Gehäusewand im Bereich der Greifrille dünner als in den daran angrenzenden Bereichen ausgeprägt werden muss. Die variierende Wanddicke hat bei der Herstellung durch Spritzgiessen den Nachteil, dass beim Aushärten des gegossenen Gehäuses stärkere Spannungen auftreten und dass das Gehäuse nicht überall gleich schnell aushärtet. Ein weiterer Nachteil entsteht in Bezug auf das erforderliche Giesswerkzeug. Zum Giessen eines Bauteils mit einer aussenseitig umlaufenden Rille ist eine mehrteilige, radial öffnende Giessform erforderlich und das Ausformen des gegossenen Gehäuses erschwert.

Um diese Nachteile zu vermeiden, wird die Greifrille wenigstens teilweise durch wenigstens eine die Gehäusewand umgebenden Greifring gebildet. Dieser Greifring wird als separates Bauteil gefertigt und anschliessend auf die Gehäusewand aufgeschoben. Der Greifring kann auf unterschiedliche Arten an der Gehäusewand befestigt werden, z.B. durch Kleben, Ultraschall-Schweissen, durch eine formschlüssige Verbindung mittels an der Gehäusewand oder dem Greifring vorgesehenen Rastnocken, welche das Aufschnappen des Greifrings erlauben, oder auch durch eine kraftschlüssige Verbindung, bei der der Greifring mit Untermass gefertigt und anschliessend auf die Gehäusewand aufgepresst wird.

Um durch Aufbringen eines Greifrings eine umlaufende Greifrille zu erzeugen, muss die Form der Gehäusewand durch den Greifring in geeigneter Weise ergänzt bzw. verändert werden. Die einfachste Möglichkeit besteht darin, einen Greifring zu verwenden, dessen Aussenfläche bereits eine umlaufende Greifrille aufweist. Diese Variante hat jedoch den Nachteil, dass zur Herstellung eines solchen Rings eine relativ komplizierte Giessform notwendig ist. Es wird daher bevorzugt ein Greifring verwendet, der sich nicht über jene Stelle hinaus erstreckt, an der die Rille am tiefsten ist. Diese tiefste Stelle der Rille wird im folgenden auch als Rillenboden bezeichnet. Um mit einem solchen Greifring eine Rille zu erzeugen, muss dieser mit wenigstens einem weiteren Element kombiniert werden. Eine einfache Möglichkeit ist die verwendung eines weiteren Greifrings, wobei die beiden Greifringe nebeneinander angeordnet werden, so dass sich zwischen den Greifringen eine Rille befindet.

Auf den zweiten Greifring kann z.B. auch verzichtet werden, wenn die Aussenfläche der Gehäusewand eine Form aufweist, die dazu geeignet ist, zusammen mit einem Greifring eine umlaufende Rille zu bilden. Ein Beispiel hierfür ist die Verwendung einer konischen Gehäusewand. Wird auf einem Konus ein Ring angeordnet, so entsteht zwischen dem Ring und der ansteigenden Konusfläche eine Rille.

Bei einer verbreitet eingesetzten Gehäuseform weist die Gehäusewand an ihrem oberen, durch den Deckel verschlossenen Ende einen radial auswärts abstehenden Flanschring auf. Auch dieser Flanschring eignet sich in Kombination mit einem Greifring zur Bildung einer Greifrille. Der Greifring wird dabei unterhalb des Flanschrings angeordnet, wobei sich der Rillenboden zwischen dem Flanschring und dem Greifring befindet.

Der Greifring weist eine Fläche auf, die gegen eine durch den Rillenboden verlaufende Ebene gerichtet ist. Diese Fläche bildet somit einen Teil der Greifrille und wird daher auch als Greiffläche bezeichnet. Bei einer bevorzugten Ausführungsvariante ist die Greiffläche unter einem Winkel von wenigstens 45° zu der durch den Rillenboden verlaufenden Ebene geneigt, wobei die Breite der Greifrille nach aussen hin, das heisst mit geringerer Tiefe der Rille, zunimmt. Eine solche Rille lässt sich im Vergleich zu einer Rille mit parallelen Seitenflächen leichter und sicherer greifen. Bevorzugt wird die Greifläche des Greifrings unter einem Winkel von 60° bis 80° zu der durch den Rillenboden verlaufenden Ebene geneigt.

Die Handhabung der Vorrichtung lässt sich weiter erleichtern, wenn die Greifläche des Greifrings geriffelt oder auf andere Art aufgerauht wird, so dass beim Hantieren mit dem Gehäuse ein Abrutschen praktisch ausgeschlossen ist.

Besonders vorteilhaft ist die erhöhte Griffsicherheit des Gehäuses bei Vorrichtungen, bei denen wenigstens eine Anschlussöffnung des Gehäusebodens und/oder des Gehäusedeckels am Ende eines zum Anschliessen eines Kunststoff-Schlauchs vorgesehenen Anschlussrohrs angeordnet ist. Solche Anschlussrohre werden zum Herstellen von Schlauchverbindungen häufig eingesetzt. Im Vergleich mit anderen Kupplungssystemen erfordert es einen relativ grossen Kraftaufwand, einen Kunststoffschlauch fest auf ein solches Anschlussrohr aufzuschieben. Bei der Verwendung dieses Kupplungssystems bringt die vorstehend beschriebene Greifrille daher besondere Vorteile.

Bei der Wahl des Kunststoffs, aus dem das Gehäuse gefertigt wird, muss auf medizinische Anforderungen Rücksicht genommen werden. Dagegen kann das Material des Greifrings frei gewählt werden, der Greifring kann insbesondere aus einem beiliebig gefärbten Kunststoff bestehen. Unterschiedlich gefärbte Greifringe können zur Unterscheidung von ansonsten gleich aussehenden Vorrichtungen verwendet werden, welche zum Beispiel unterschiedliche Behandlungs-Einsätze enthalten. Der Greifring wird bevorzugt aus einem thermoplastischen Kunststoff gefertigt, da sich dieser besonders leicht verarbeiten lässt und der Greifring so eine gewisse Elastizität erhält.

Die Erfindung ist im folgenden anhand von Ausführungsbeispielen näher erläutert. Es zeigen:
- Figur 1: die Schnittdarstellung einer Vorrichtung zur Atemluft-Behandlung,
- Figur 2: die Ansicht der in Figur 1 gezeigten Vorrichtung aus der Sicht von unten,
- Figur 3: die Schnittdarstellung einer alternativen Ausführungsvariante mit zwei im Abstand voneinander angeordneten Greifringen und
- Figur 4: die Schnittdarstellung einer Vorrichtung mit einem Greifring, der in Kombination mit einem Flansch der Gehäusewand eine Greifrille bildet,
- Figur 5: ein Ausführungsbeispiel analog Figur 4 im Teilschnitt.

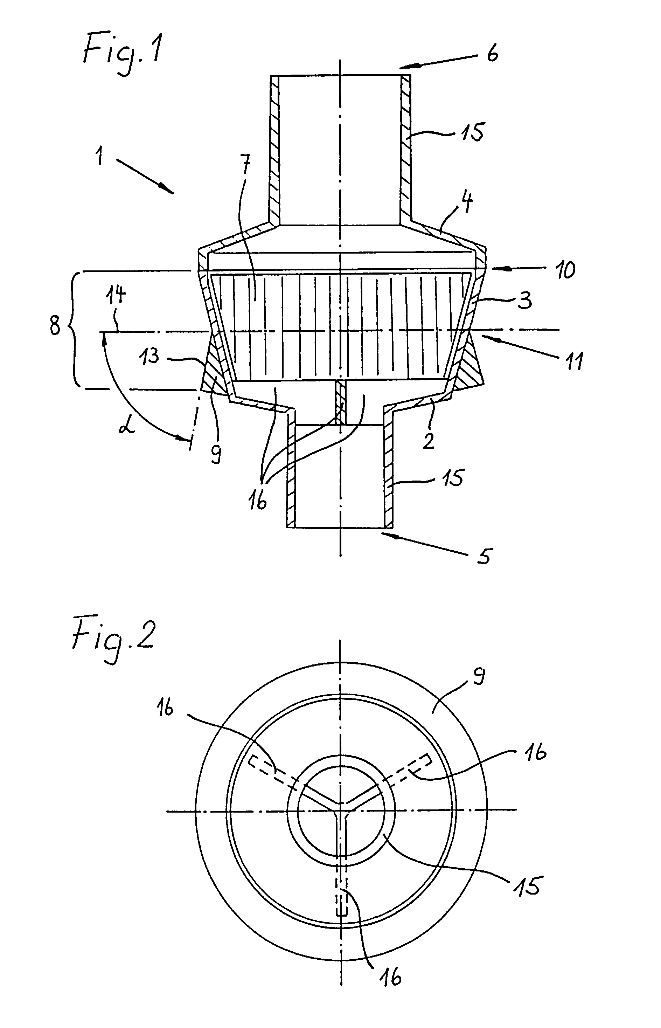

Das Kunststoff-Gehäuse 1 der in Figur 1 im Schnitt dargestellten Vorrichtung zur Atemluft-Behandlung weist einen unteren Teil mit einem Gehäuseboden 2 und einer Gehäusewand 3 auf, welche sich ausgehend vom Gehäuseboden 2 rohrförmig nach oben erstreckt. An ihrem oberen Ende 10 ist die Gehäusewand 3 durch einen Deckel 4 verschlossen. Eine erste Anschlussöffnung 5 ist im Gehäuseboden 2 vorgesehen und eine zweite Anschlussöffnung 6 in dem Deckel 4. Die Anschlussöffnung 5, 6 sind je am Ende eines Anschlussrohrs 15 angeordnet, wobei die Anschlussrohre 15 im Sinne der hier verwendeten Terminologie mit zum Gehäuseboden 2 bzw. dem Deckel 4 gehören. Die Anschlussrohre 15 sind zur Befestigung von Schlauchenden vorgesehen, welche zu diesem Zweck über die Anschlussrohre geschoben werden (nicht gezeichnet).

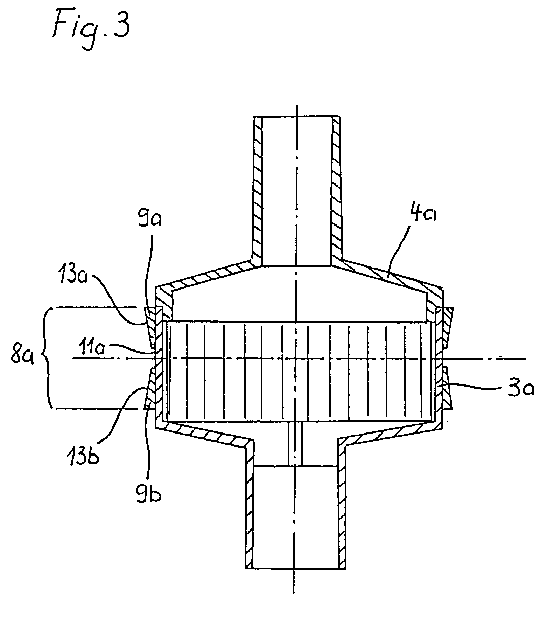

Der Deckel 4 ist durch Ultraschall-Schweissen fest mit der Gehäusewand 3 verbunden. In dem Gehäuse ist ein Behandlungs-Einsatz 7 angeordnet, dessen untere Fläche auf drei sternförmig angeordneten Rippen 16 des Gehäuses aufliegt. Diese Rippen sind auch in Figur 2 deutlich erkennbar, in der dieselbe Vorrichtung in einer Ansicht von unten dargestellt ist.

Die Aussenfläche des Gehäuses weist eine umlaufende Rille 8 auf, wobei die obere Hälfte der Rillenfläche durch die konische Gehäusewand 3 und die untere Hälfte durch die Greiffläche 13 des auf die Gehäusewand aufgeschobenen Greifrings 9 gebildet wird. Die als Rillenboden bezeichnete, tiefste Stelle 11 der Rille befindet sich bei diesem Ausführungsbeispiel am oberen Rand des Greifrings 9. Zu der durch den Rillenboden 11 verlaufenden Ebene 14 ist die Greiffläche 13 unter einem Winkel von etwa 80° geneigt. Durch diese starke Neigung ergibt sich eine relativ flache Rille, die sich leicht greifen lässt.

Figur 3 zeigt ein alternatives Ausführungsbeispiel, bei dem die Gehäusewand 3a eine zylindrische Aussenfläche aufweist. Auf diese Gehäusewand 3a sind zwei Greifringe 9a, 9b aufgeschoben und im Abstand zueinander angeordnet. Da die Aussenflächen 13a, 13b der Greifringe 9a, 9b leicht zueinander geneigt sind, könnte auch auf den Abstand zwischen den beiden Ringen 9a, 9b verzichtet werden und es würde dennoch eine Greifrille bestehen. In diesem Fall könnte anstelle der beiden einzelnen Greifringe auch ein einzelner, entspreched geformter Greifring verwendet werden, dessen Herstellung jedoch wesentlich aufwendiger wäre.

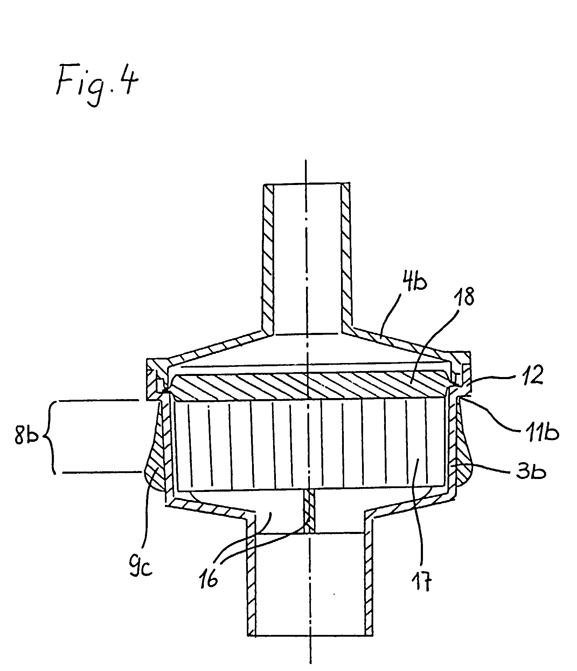

Figur 4 zeigt die Schnittdarstellung eines weiteren Ausführungsbeispiels, bei dem die Gehäusewand 3b etwa zylindrisch ist und an ihrem oberen, durch den Deckel 4b verschlossenen Ende einen radial auswärts abstehenden Flanschring 12 aufweist. Die zylindrische Gehäuseform wird bevorzugt verwendet, da der in das Gehäuse eingesetzte Feuchtigkeits- und Wärmetauscher 17 vorzugsweise aus gewickeltem, gerilltem Papier besteht und somit von Natur aus eine zylindrische Aussenfläche aufweist. Die Stege 16, auf denen der Feuchtigkeits- und Wärmetauscher aufliegt verhindern, dass Teile des gewikkelten Papiers durch die Luftströmung in den Bereich der Anschlussöffnung oder gar in das daran angeschlossene Schlauchsystem geraten. In diesem Beispiel ist zusätzlich zu dem Feuchtigkeits- und Wärmetauscher ein Filterflies 18 eingesetzt, welches primär Staubpartikel und Bakterien zurückhalten soll.

Der Flanschring 12 bildet zusammen mit einem unterhalb des Flanschrings angeordneten Greifring 9c eine Greifrille 8b. Der Rillenboden 11b befindet sich dabei ganz am oberen Rand der Rille 8b, welche mit dem oberen Rand des Greifrings 9c zusammenfällt.

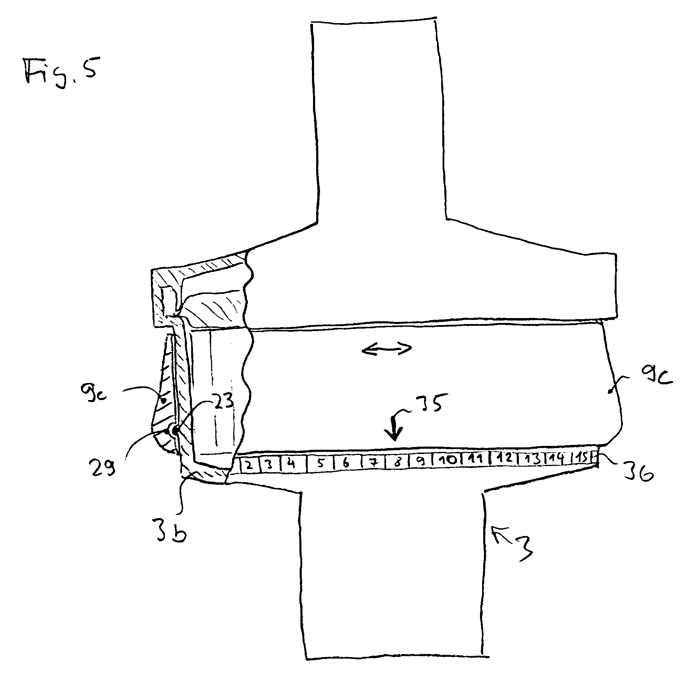

Figur 5 zeigt ein Ausführungsbeispiel analog Figur 4 im Teilschnitt. Der Greifring 9c weist dabei an seiner inneren, der Gehäusewand 3b zugewandten Oberfläche eine ringförmige Vertiefung 29 auf. In die Vertiefung 29 ragt ein ringförmiger Vorsprung 23, der von der Oberfläche der Gehäusewand 3b absteht. Der Greifring 9c weist einen etwas grösseren Innendurchmesser auf als der Aussendurchmesser der Gehäusewand 3b, so dass der Greifring 9c mit geringem Kraftaufwand relativ zur Gehäusewand 3b verdreht werden kann. Durch die Rille 29 und den Vorsprung 23 wird der Greifring 9c dabei aber unverlierbar auf der Gehäusewand 3b gehalten. Auf der Oberfläche des Greifrings 9c ist als Markierungselement ein Pfeil 35 angebracht. Unterhalb des Greifrings 9c ist auf der Oberfläche der Gehäusewand 3b ein Markierungsfeld 36 vorgesehen. Durch Verdrehen des Greifrings 9c relativ zur Gehäusewand 3b lässt sich der Pfeil 35 zur Deckung mit einem Zeichen des Markierungsfeldes 36 bringen. Beim Ausführungsbeispiel gemäss Figur 5 trägt das Markierungsfeld Datums-Zahlen. Das Pflegepersonal kann beim ersten Einsatz den Pfeil 35 auf die aktuelle Datums-Zahl im Markierungsfeld 36 stellen. Damit lässt sich jederzeit ablesen, wie lange ein derartiger Filter im Einsatz war und ob z.B. ein Austausch vorgenommen werden muss. Statt der Datumsanzeige lassen sich auch andere Daten im Markierungsfeld 36 vorsehen. Die Anordnung lässt sich auch beliebig ändern, so z.B. durch Anbringen eines Fenster-Ausschnitts im Greifring 9c, der jeweils darunterliegende Datenfelder beim Verdrehen selektiv zur Anzeige bringt. Werden zwei Greifringe vorgesehen, lassen sich ersichtlicherweise verschiedene Datenfelder vorsehen. Es wäre auch denkbar, den Greifring nicht nur verdrehbar, sondern auch vertikal verlagerbar, z.B. in unterschiedlichen Raststellungen einschnappbar zu gestalten, um sowohl durch vertikale Verlagerung als auch durch Verdrehbewegung entsprechende Anzeige vorzunehmen.

## Patentansprüche

1. Vorrichtung zur Behandlung von Atemluft, mit einem etwa zylindrischen Kunststoffgehäuse (1) mit einem Gehäuseboden (2), einer Gehäusewand (3) und einem Deckel (4), wobei im Gehäuseboden (1) und im Gehäusedeckel (4) je wenigstens eine Anschluss-Oeffnung (5, 6) vorgesehen ist und in dem Gehäuse wenigstens ein Behandlungs-Einsatz (7) angeordnet ist, dadurch gekennzeichnet, dass die Aussenfläche des Gehäuses eine entlang dem Umfang der Gehäusewand (3) verlaufende Greifrille (8) aufweist, die wenigstens teilweise durch wenigstens einen die Gehäusewand umgebenden Greifring (9) gebildet wird.

2. Vorrichtung nach Anspruch 1, dadurch gekennzeichnet, dass die Greifrille (8) einen Rillenboden (11) aufweist, in dessen Bereich die Rille (8) am tiefsten ist, wobei sich der Greifring (9) nicht über den Rillenboden (11) hinaus erstreckt, so dass der Greifring (9) selbst keine Rille aufweist.

3. Vorrichtung nach Anspruch 2, dadurch gekennzeichnet, dass die Greifrille (8a) durch zwei nebeneinander angeordnete Greifringe (9a, 9b) gebildet wird.

4. Vorrichtung nach Anspruch 2, dadurch gekennzeichnet, dass die Gehäusewand (3b) an ihrem oberen, durch den Deckel (4b) verschlossenen Ende einen radial auswärts abstehenden Flanschring (12) aufweist und die Greifrille (8b) durch diesen Flanschring (12) und einen unterhalb des Flanschrings angeordneten Greifring (9c) gebildet wird.

5. Vorrichtung nach einem der Ansprüche 2 bis 4, dadurch gekennzeichnet, dass der Greifring (9) eine Greiffläche (13) aufweist, die gegen eine durch den Rillenboden (11) verlaufende Ebene (14) gerichtet ist, wobei die Greiffläche (13) zu der genannten Ebene (14) unter einem Winkel (α) von wenigstens 45° derart geneigt ist, dass die Breite der Greifrille (8) nach aussen hin zunimmt.

6. Vorrichtung nach Anspruch 5, dadurch gekennzeichnet, dass die Greiffläche (13) unter einem Winkel (α) von 60° bis 80° zu der durch den Rillenboden verlaufenden Ebene (14) geneigt ist.

7. Vorrichtung nach einem der Ansprüche 5 oder 6, dadurch gekennzeichnet, dass die Greiffläche (13) eine Riffelung aufweist.

8. Vorrichtung nach einem der Ansprüche 1 bis 7, dadurch gekennzeichnet, dass wenigstens eine Anschluss-Oeffnung (5, 6) des Gehäusebodens (2) und/oder des Gehäusedeckels (4) am Ende eines zum Anschliessen eines Kunststoff-Schlauchs vorgesehenen Anschlussrohrs (15) angeordnet ist.

9. Vorrichtung nach einem der Ansprüche 1 bis 8, dadurch gekennzeichnet, dass der Kunststoff des Greifrings gefärbt ist.

10. Vorrichtung nach einem der vorangegangenen Ansprüche, dadurch gekennzeichnet, dass der Greifring (9) relativ zum Gehäuse (3) verlagerbar, insbesondere verdrehbar ist.
